Europäisches Patentamt

European Patent Office

Office européen des brevets

(19)

(11) Numéro de publication: **0 304 344**
**A1**

(12) **DEMANDE DE BREVET EUROPEEN**

(21) Numéro de dépôt: 88401293.1

(22) Date de dépôt: 26.05.88

(51) Int. Cl.⁴: **A 61 K 39/29**
G 01 N 33/576

(30) Priorité: 26.05.87 FR 8707411

(43) Date de publication de la demande:
22.02.89 Bulletin 89/08

(84) Etats contractants désignés:
AT BE CH DE ES FR GB GR IT LI LU NL SE

(71) Demandeur: INSTITUT NATIONAL DE LA SANTE ET DE
LA RECHERCHE MEDICALE (INSERM)
101, rue de Tolbiac
F-75013 Paris (FR)

(72) Inventeur: **Coursaget Pierre**
6, Place de Richemont
37170 - Saint-Avertin (FR)

**Yvonnet Bernard**
253 bis rue E. Vaillant
37000 Tours (FR)

**Bourdil Claude**
44, rue Elise Dreux
37000 Tours (FR)

(74) Mandataire: **Gutmann, Ernest et al**
S.C. Ernest Gutmann - Yves Plasseraud 67, boulevard
Haussmann
F-75008 Paris (FR)

Le titre de l'invention a été modifié (Directives relatives à l'examen pratiqué à l'OEB, A-III, 7.3)

(54) **Virus apparenté au virus de l'hépatite B, ses constituants et leur application comme vaccin ou réactif de diagnostic.**

(57) L'invention concerne des particules d'un virus apparenté au virus de l'hépatite B, dont les enveloppes sont reconnues par des anticorps polyclonaux anti-AgHBs, dont les nucléocapsides sont distinctes de celles du virus de l'hépatite B, ces particules virales présentent une densité d'environ 1,25 à environ 1,275 dans un gradient de chlorure de coesium et des diamètres mesurables en microscopie électronique de 45 à 60 nm. Les enveloppes de ces virus, lorsqu'elles sont dépourvues d'ADN viral, ont des diamètres d'environ 22 nm à environ 30 nm.

Les enveloppes de ces virus sont utiles pour le diagnostic d'hépatites et pour la vacination contre celles-ci.

EP 0 304 344 A1

Description

## VIRUS APPARENTE AU VIRUS DE L'HEPATITE VIRALE B, SES DIVERS CONSTITUANTS ET LEUR APPLICATION AU DIAGNOSTIC D'UNE HEPATITE VIRALE ET A LA VACCINATION CONTRE CETTE MALADIE

Lors d'une étude de protection à long terme contre l'hépatite B, réalisée fin 1985 et 1986 chez des enfants, il a été observé chez de très nombreux porteurs d'antigènes HBs (AgHBs positifs) la particularité suivante :

1. Aucun ne possédait d'anticorps anti-HBc ;

2. Une majorité de ces porteurs avaient été vaccinés contre l'hépatite B et possédaient toujours les anticorps protecteurs anti-HBs (parfois à des titres très élevés) ;

3. Pour la majorité des sujets prélevés deux à six mois plus tard, il y avait eu disparition de l'AgHBs et jamais d'apparition d'anticorps anti-HBc, ni d'anticorps anti-HBs. Les autres sont restés porteurs de l'AgHBs, sans apparition d'anticorps anti-HBc.

4. Aucun de ces sujets n'avait présenté de maladie au sens clinique du terme.

Il a d'abord été envisagé de considérer que ces résultats consistaient en de faux positifs, dus à une erreur de manipulation, soit lors des prélèvements, soit à l'occasion du traitement des sérums ou des tests.

Toutefois, une enquête devait montrer que toutes les opérations effectuées sur les sérums avaient été réalisées dans de bonnes conditions. De plus, il convenait d'exclure la possibilité que peut-être des contaminations entre sérums étaient intervenues, puisqu'aucun sérum concerné ne contenait d'anticorps anti-HBc.

Pour tous ces sujets AgHBs positifs, des tests radio immunologiques et immunoenzymatiques ont été refaits. Les résultats ont été confirmés dans un essai radio-immunologique (RIA) et par une méthode ELISA pour tous les sujets ayant un S/N > 10 (le rapport S/N correspondant au rapport de cpm (coups par minute) de 1 échantillon/cpm des témoins négatifs). Puis les essais ont été répétés à l'aide d'une batterie d'anticorps monoclonaux dirigés contre plusieurs épitopes spécifiques portés par les antigènes du virus de l'hépatite B, sur des sérums provenant à la fois de porteurs désignés dans les paragraphes 2. et 3. ci-dessus.

Ces essais ont été plus particulièrement réalisés avec des anticorps monoclonaux reconnaissant l'épitope "a" se situant au niveau des antigènes HBs de tous les soustypes de virus de l'hépatite B, et avec des anticorps monoclonaux reconnaissant une partie du peptide pré-S2 (codé par le gène pré-S2 situé en amont du gène S dans le génome des virus de l'hépatite B). Pour une meilleure compréhension de ce qui suit, il faut souligner que le génome des virus de l'hépatite B comprend le gène S codant pour la "protéine majeure", en amont duquel se trouve le gène pré-S2 codant en combinaison avec le gène S pour la "protéine-moyenne", le gène pré-S2 étant lui-même précédé par le gène pré-S1 codant en combinaison avec les gènes pré-S2 et S pour la "grande protéine" ; si l'on considère cette "grande protéine" constituée successivement par les parties peptidiques codées par les gènes pré-S1, pré-S2, et S, le premier acide aminé N-terminal situé dans la partie pré-S1 correspond à l'acide aminé numéro 1, et le dernier acide aminé C-terminal situé dans la partie S correspond à l'acide aminé 386 (ou 400 en fonction du sous-type du virus considéré), la région pré-S2 étant délimitée par les acides aminés correspondant aux positions 120 du côté N-terminal et 174 du côté C-terminal.

Parmi les anticorps monoclonaux (ACM) sus-mentionnés, on citera, pour ceux de spécificité "a", les ACM 15A7 et 144A2 décrits dans COUROUCE et al, Symposium on Virus Hepatitis, Athène, GRECE, (1982) - Development in Biological Standardization, 54, 527-534 (1983), et pour ceux de spécificité pré-S2, les ACM F376 (MICHEL et al, PNAS, 81, 7708-7712 (1984)), et F124 (PETIT et al, Journal of General Virology, 68, 2759-2767 (1987)). L'ACM F376 reconnait spécifiquement une séquence d'acides aminés comprise entre les acides aminés correspondant aux positions 132 et 140 de la partie pré-S2 (NEURATH et al, Molecular Immunology, 23, 991-997 (1986)). L'ACM F124 reconnait les deux tiers environ de la région N-terminale de la partie pré-S2 (lorsque cette dernière se présente sous une conformation favorable), et plus particulièrement la séquence d'acides aminés comprise entre les acide aminés correspondant aux positions 120 et 150.

Les études réalisées à l'aide des ACM sus-mentionnées ont conduit les Inventeurs à déterminer que les ACM F376 et 15A7 ne reconnaissant pas les sérums des porteurs sus-mentionnés, tandis que les ACM F124 et 144A2 reconnaissant lesdits sérums.

Il s'avère donc que l'enveloppe des particules virales contenues dans les sérums des patients sus-mentionnés ne porte qu'une partie de la spécificité S et qu'une partie de la spécificité pré-S2.

En outre, l'examen en microscopie électronique et en immuno-microscopie du sérum de certains des porteurs d'AgHBs allait révéler la présence de particules virales caractéristiques et des particules plus petites correspondant à des fragments d'enveloppe de ces virus. Ces différentes particules ont été purifiées par les techniques couramment appliquées pour l'isolement et la purification des particules soit du virus de l'hépatite B, soit de l'antigène AgHBs à partir des sérums qui normalement les contiennent. L'étude approfondie de ces particules a conduit à la conclusion que l'on se trouvait en présence de virus distincts du virus de l'hépatite B, qui n'en possédaient pas moins certaines des caractéristiques de ce dernier.

L'invention concerne donc plus particulièrement ces nouvelles particules virales et leurs constituants. Les particules virales de base peuvent être définies de la façon suivante :

- leurs enveloppes sont reconnues par des anticorps polyclonaux anti-AgHBs ;

- les nucléocapsides de ces particules virales sont distinctes de celle du virus de l'hépatite B, cette différence se manifestant par l'absence de détection d'anticorps anti-AgHBc et d'anticorps anti-AgHBe chez un hôte infecté par ces particules virales,

- ces particules virales présentent une densité d'environ 1,25 à environ 1,275 dans un gradient de chlorure de coesium et des diamètres mesurables en microscopie électronique de 45 à 60 nm ;

- leurs enveloppes dépourvues d'ADN viral ont des diamètres d'environ 22 nm à environ 30 nm ;

- ces particules virales sont reconnues par un anticorps monoclonal reconnaissant spécifiquement une séquence d'acides aminés comprise entre les acides aminés correspondant aux positions 120 et 150 du peptide pré-S2 ;

- elles ne sont pas reconnues par un anticorps monoclonal reconnaissant spécifiquement une séquence d'acides aminés comprise entre les acides aminés correspondant aux positions 132 et 140 du peptide pré-S2 ;

Le fait que ces particules virales selon l'invention ne soient pas reconnues par la totalité des anticorps monoclonaux de spécificité "a" confirme également que lesdites particules sont des virus responsables d'une hépatite et différents des virus de l'hépatite B.

En particulier, il a été observé que ce virus pouvait n'induire jamais in vivo d'anticorps anti-HBsAg (donc des anticorps contre l'antigène d'enveloppe du virus de l'hépatite B).

Les particules selon l'invention sont en général également caractérisées par leur caractère infectieux chez l'hôte vis-à-vis de sujets vaccinés contre l'hépatite B, qui sont porteurs d'anticorps anti-AgHBs. En particulier elles paraissent conserver leur caractère infectieux vis-à-vis de sujets qui ne sont pas porteurs d'anticorps anti-préS2.

L'invention concerne plus particulièrement encore des particules qui induisent chez l'hôte un processus de développement impliquant non seulement l'absence d'anticorps anti-AgHBc et d'anticorps AgHBe, mais aussi le nondéveloppement à terme des anticorps anti-AgHBs, notamment lorsque l'hôte atteint le stade de la guérison définitive.

Ces observations entraînent la capacité pour ces particules d'induire une hépatite même chez des personnes vaccinées avec un vaccin hépatite B contenant la protéine HBs du virus de l'hépatite B. Cette activité peut être vérifiée par un essai d'infection chez le chimpanzé préalablement vacciné avec le susdit vaccin de l'hépatite B, cet essai devant alors avoir pour effet l'induction d'une hépatite biologique. De même l'inoculation de chimpanzés avec des enveloppes du virus de l'invention doit conduire à la détection d'anticorps caractéristiques des particules virales selon l'invention, en ce qu'ils sont reconnus par ceux des anticorps polyclonaux ou monoclonaux susmentionnés contre des épitopes du virus de l'hépatite B qui reconnaissent aussi les particules virales selon l'invention et ne sont pas reconnus par ceux des anticorps monoclonaux mentionnés plus haut et dirigés contre d'autres épitopes de l'hépatite B qui ne reconnaissent pas les mêmes particules virales.

Ces particules virales, préalablement débarrassées de leurs ADNs, et plus particulièrement les particules d'enveloppe dont les tailles sont légèrement plus élevées que celles des particules d'antigène AgHBs du virus de l'hépatite B, constituent donc des principes actifs de choix pour la constitution de principes actifs de vaccins contre cette forme atypique de virus de l'hépatite B et éventuellement aussi les hépatites virales B les plus répandues.

Dans une variante de l'invention, les principes vaccinants dérivables du virus selon l'invention peuvent être utilisés en association avec les principes actifs correspondants dérivés du virus de l'hépatite B, en particulier de l'antigène AgHBs, de façon à fournir une composition de vaccin ayant un spectre ou une efficacité de protection plus étendu contre les hépatites virales B ou celles qui leur sont apparentes.

L'invention concerne également l'utilisation des différents antigènes susceptibles d'être obtenus à partir de ce virus atypique par les mêmes techniques que celles appliquées au virus de l'hépatite B ou à l'antigène AgHBs, pour la fabrication de compositions de diagnostic permettant l'identification plus précise des anticorps éventuellement présents dans des sérums ou tissus de patients à l'étude, et plus particulièrement d'anticorps reconnaissant les particules virales qui ont été définies plus haut.

L'invention concerne également encore les ADNs susceptibles d'être isolés à partir de ces virus et leurs applications à la constitution de sondes d'hybridation permettant la caractérisation plus fine des ADNs contenus dans des sérums de patients, chez lesquels une hépatite a été détectée. Les techniques utilisées pour l'isolement de ces ADNs, leur clonage éventuel dans des vecteurs, notamment des plasmides appropriés dans le domaine de l'hépatite B sont directement transposables à l'isolement et au clonage des ADNs des particules virales selon la présente invention. Il en est également de même des conditions dans lesquelles les essais d'hybridation mettant en oeuvre ces sondes doivent être effectués.

L'invention concerne par conséquent également les différentes séquences d'ADNs codant pour les peptides portant des épitopes caractéristiques susceptibles d'être mis en oeuvre soit pour le diagnostic, soit dans la constitution de vaccins susceptibles de conférer aux sujets les recevant une immunité les protégeant contre le virus atypique selon l'invention et, le cas échéant aussi, plus généralement contre les virus de l'hépatite B.

Il a été confirmé que le virus selon l'invention peut être identifié chez une proportion importante d'individus sujets à une hépatite virale, comme le montre la lettre publiée dans Lancet du 30 avril 1988, "Hepatitis B Virus Type II" n° 8592, page 990.

## Revendications

1. Particules virales, caractérisées par la combinaison de propriétés suivantes :

- leurs enveloppes sont reconnues par des anticorps polyclonaux anti-AgHBs ;

- les nucléocapsides de ces particules virales sont distinctes de celles du virus de l'hépatite B, cette différence se manifestant par l'absence de détection d'anticorps anti-AgHBc et d'anticorps anti-AgHBe chez un hôte infecté par ces particules virales ;

- ces particules virales présentent une densité d'environ 1,25 à environ 1,275 dans un gradient de chlorure de coesium et des diamètres mesurables en microscopie électronique de 45 à 60 nm ;

- leurs enveloppes dépourvues d'ADN viral ont des diamètres d'environ 22 nm à environ 30 nm.

- ces particules virales sont reconnues par un anticorps monoclonal reconnaissant spécifiquement une séquence d'acides aminés comprise entre les acides aminés correspondant aux positions 120 et 150 du peptide pré-S2 ;

- elles ne sont pas reconnues par un anticorps monoclonal reconnaissant spécifiquement une séquence d'acides aminés comprise entre les acides aminés correspondant aux positions 132 et 140 du peptide pré-S2 ;

2. Particules virales selon la revendication 1, caractérisées par le fait que leurs enveloppes ne portent qu'une partie de la spécificité S et qu'une partie de la spécificité pré-S2.

3. Particules selon la revendication 1 ou la revendication 2, caractérisées en ce qu'elles restent infectieuses vis-à-vis de sujets vaccinés contre l'hépatite B, qui sont porteurs d'anticorps anti-AgHBs.

4. Particules selon la revendication 3, caractérisées en ce qu'elles restent infectieuses vis-à-vis de sujets vaccinés qui ne sont pas porteurs d'anticorps anti-préS2.

5. Particules virales selon la revendication 4, caractérisées par leur caractère infectieux chez l'hôte et par un processus de développement chez l'hôte infecté conduisant à la préservation chronique chez l'hôte de sa capacité à synthétiser des anticorps anti-prés2, à l'exclusion d'anticorps anti-AgHBc et anti-AgHBe.

6. Particules virales selon la revendication 5, caractérisées en ce qu'elles induisent chez l'hôte un processus de développement impliquant la non-détection des anticorps anti-AgHBs, lorsque l'hôte a atteint le stade de la guérison.

7. Particules selon l'une quelconque des revendications 1 à 6, caractérisées en ce qu'elles sont isolable à partir d'êtres humains qui sont porteurs d'antigènes HBs, en l'absence d'anticorps anti-HBc.

8. Particules dépouvues d'acides nucléiques viraux et caractérisées par la combinaison de propriétés suivantes :

- elles sont reconnues par des anticorps polyclonaux anti-AgHBs ;

- elles sont exemptes d'antigènes anti-AgHBc et AgHBe ;

- elles ont des diamètres d'environ 22 nm à environ 30 nm;

- elles sont immunogènes, mais n'induisent pas in vivo d'anticorps anti-HBs, anti-HBc et anti-HBe.

9. Particules virales selon la revendication 8, caractérisées par le fait qu'elles ne portent qu'une partie de la spécificité S et qu'une partie de la spécificité pré-S2, comme l'atteste l'incapacité de tous les anticorps monoclonaux formés contre les différentes parties des peptides porteurs des spécificités correspondantes du virus de l'hépatite B à réagir avec les antigènes d'enveloppe des susdites particules virales.

10. Particules selon la revendication 8 ou la revendication 9, caractérisées par le fait qu'elles comportent une partie au moins de la spécificité pré-S2.

11. Composition de vaccin contenant des particules selon l'une quelconque des revendications 8 à 10.

12. Procédé de diagnostic in vitro de la présence dans un sérum d'anticorps contre les particules virales selon l'une quelconque des revendications 1 à 7, comprenant la mise en contact d'un antigène consistant en des particules selon l'une des revendications 8 à 10 ou des peptides normalement exposés à la surface de ces particules avec le sérum à doser et la détection du complexe immunologique formé entre ces anticorps et ledit antigène.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | WO-A-8 401 107 (THE GENERAL HOSPITAL CORP.) <br> * En entier, en particulier page 3, ligne 17 - page 4, ligne 16; page 13, ligne 17 - page 15, ligne 15; page 24, ligne 10 - page 26, ligne 1; page 30, lignes 8-23,28-32; page 33, ligne 1 - page 35, ligne 22; page 42, tableau 1; page 44, ligne 25 - page 45, ligne 21; page 50, tableau 4; page 52, lignes 1-7,28-31; pages 54-56; page 57, lignes 22-31; revendications * | 1-12 | A 61 K 39/29 <br> G 01 N 33/576 |
| A,O | J. MED. VIROL., vol. 21, no. 4, 1987, page 13A, no. 36; V. THIERS et al.: "Transmission of HBV and related viruses from HBsAg negative, HBV DNA and monoclonal anti-Hbs positive, human sera to chimpanzees" <br> * Résumé * | 1 | |
| A,O | J. MED. VIROL., vol. 21, no. 4, 1984, pages 46A-47A, no. 133; M. LALIAM et al.: "Detection of hepatitis B virus DNA and HBsAg (polyclonal and monoclonal anti-HBs radioimmunoassays) in sera of Algerian children with acute non-A, non-B hepatitis" <br> * Résumé * <br> ---       -/- | 1 | **DOMAINES TECHNIQUES RECHERCHES (Int. Cl.4)** <br><br> A 61 K |

Le présent rapport a été établi pour toutes les revendications

| Lieu de la recherche | Date d'achèvement de la recherche | Examinateur |
|---|---|---|
| LA HAYE | 28-09-1988 | SKELLY J.M. |

## RAPPORT DE RECHERCHE EUROPEENNE

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | BIOLOGICAL ABSTRACTS, vol. 82, 1986, no. 94314, Philadelphia, US; M.C. KEW et al.: "Comparison between polycloman and first and second generation monoclonal radioimmunoassays in the detection of hepatitis B surface antigen in patients with hepatocellular carcinoma", & HEPATOLOGY (BALTIMORE) 6(4): 636-639, 1986 * Résumé * | | |
| A | BIOLOGICAL ABSTRACTS, vol. 81, 1985, no. 33667, Philadelphia, US; E. BEN-PORATH et al.: "Structural analysis of hepatitis B surface antigen by monoclonal antibodies", & J. CLIN. INVEST. 76(4): 1338-1347, 1985 * Résumé * | | |
| A | LA RECHERCHE, vol. 14, no. 145, juin 1983, pages 854-865, Paris, FR; A. ZOTOV: "Les hépatites" | | **DOMAINES TECHNIQUES RECHERCHES (Int. Cl.4)** |
| E | EP-A-0 279 460 (SEELIG) * Revendications * | 12 | |
| P,X | LANCET, vol. II, no. 8572, 12 décembre 1987, pages 1354-1358; P. COURSAGET et al.: "HBsAG positive reactivity in man not due to hepatitis B virus" * En entier * | 1-12 | |

Le présent rapport a été établi pour toutes les revendications

| Lieu de la recherche | Date d'achèvement de la recherche | Examinateur |
|---|---|---|
| LA HAYE | 28-09-1988 | SKELLY J.M. |